# EUROPEAN PATENT SPECIFICATION

(11) **EP 1 524 274 B1**
(45) Date of publication and mention of the grant of the patent: **05.03.2008**
(21) Application number: 04024029.3
(22) Date of filing: 08.10.2004
(51) Int. Cl.: C07K 14/60, G01N 33/74

(54) **Fluorescently labelled Ghrelin peptides.**
Fluoreszent markierte Ghrelin-Peptide
Peptides derivés de Ghrelin renfermant des marqueurs fluorescents

(30) Priority: 16.10.2003 EP 03023568
(43) Date of publication of application: 20.04.2005
(73) Proprietor: F. HOFFMANN-LA ROCHE AG, 4070 Basel (CH)
(72) Inventor: Enderle, Thilo, 79618 Rheinfelden (DE); Graf, Martin, 4314 Zeiningen (CH); Hertel, Cornelia, 4142 Muenchenstein (CH); Zoffmann, Sannah Jensen, 4058 Basel (CH); Kitas, Eric Argirios, 4147 Aesch (CH)

(56) References cited:
- WO-A2-01/38355
- WO-A2-01/92292
- BULLOK KRISTIN E ET AL: "Characterization of novel histidine-tagged Tat-peptide complexes dual-labeled with 99mTc-tricarbonyl and fluorescein for scintigraphy and fluorescence microscopy." BIOCONJUGATE CHEMISTRY, vol. 13, no. 6, 18 October 2002 (2002-10-18), pages 1226-1237, XP002315750 ISSN: 1043-1802
- LICHER T ET AL: "The coupling region of F0F1 ATP synthase: binding of the hydrophilic loop of F0 subunit c to F1" FEBS LETTERS, ELSEVIER SCIENCE PUBLISHERS, AMSTERDAM, NL, vol. 431, no. 3, 24 July 1998 (1998-07-24), pages 419-422, XP004258169 ISSN: 0014-5793
- BLACKMAN M J ET AL: "Structural and Biochemical Characterization of a Fluorogenic Rhodamine-Labeled Malarial Protease Substrate" BIOCHEMISTRY, AMERICAN CHEMICAL SOCIETY. EASTON, PA, US, vol. 41, 2002, pages 12244-12252, XP002239039 ISSN: 0006-2960
- MUCCIOLI G ET AL: "Binding of I25l-labeled ghrelin to membranes from human hypothalamus and pituitary gland" JOURNAL OF CLINICAL INVESTIGATION, NEW YORK, NY, US, vol. 24, 2001, pages RC7-RC9, XP002277339 ISSN: 0021-9738

## Description

Growth hormone, which is secreted from the pituitary, stimulates growth of all tissues of the body that are capable of growing. In addition, growth hormone is known to have the following basic effects on the metabolic processes of the body: (1) Increased rate of protein synthesis in all cells of the body; (2) Decreased rate of carbohydrate utilization in cells of the body; (3) Increased mobilization of free fatty acids and use of fatty acids for energy. A deficiency in growth hormone secretion can result in various medical disorders, such as dwarfism.

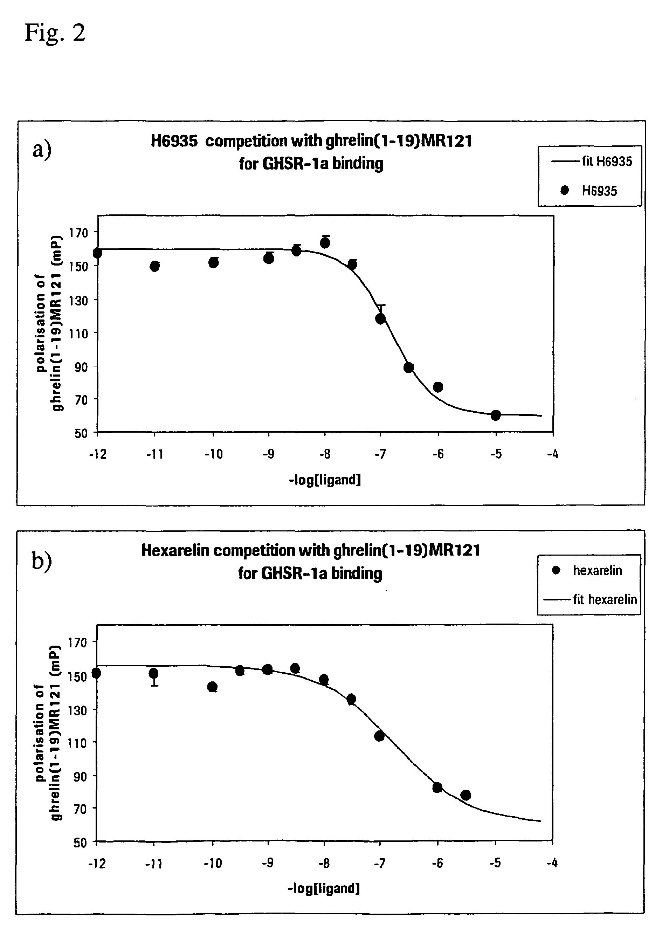

Methodology is known in the art to determine the activity of a compound as a growth hormone secretagogue. For example, an ex vivo assay is described by Smith, et al., Science, 260,1640-1643 (1993) (see text of FIG. 2 therein), but this assay requires the use of cell cultures and does not give an indication of competitive binding activity. Accordingly, it would be desirable to develop a non- radioactively labeled ligand which can be used to identify and characterize cellular receptors which play a role in the activity of growth hormone secretagogue. It would also be desirable to have a non-radioactively labeled ligand available for use in an assay for testing compounds for growth hormone secretagogue activity.

Such studies normally require a high specific activity radio-ligand. Previous attempts to develop a binding assay using [T]-labeled or [125I]-labeled peptide ligands derived from GHRP-6 met with limited success. See R. F. Walker, et al. Neuropharmacol. 989,28, 1139 and C. Y. Bowers et al., Biochem. Biophys. Res. Comm. 1991, 178,31. Generally, the binding of such peptide ligands was of low affinity and of excessively high capacity. Moreover, the binding affinities did not correlate with the growth hormone secretory activity of the peptides. The lack of correlation of binding and growth hormone secretory activity most likely was the result of the relatively low specific activity (in the case of [T] GHRP-6) and non-specific binding properties of the radio-ligands.

The problem to be solved by the present invention was to provide a fluorescent labeled ghrelin analog which can be used in high throuput screening.

The present invention pertains to a labeled growth hormone secretagogue of the formula

R1-Cys-F

wherein R1 is a peptide sequence derived from the ghrelin polypeptide sequence (Seq ID No. 1), and F is a fluorescent dye. In a preferred embodiment, R1 is Seq ID No. 2. In another preferred embodiment, R1 is octanoylated. In another preferred embodiment, R1 is N-octanoylated. In a more preferred embodiment, R1 is ¹Gly-Ser-Dapa(N-octanoyl)-Phe-⁵Leu-Ser-pro-Glu-His-¹⁰Gln-Arg-Val-Gln-Gln-¹⁵Arg-Lys-Glu-Ser. N-octanoylation increases stability of ghrelin towards esterase activity. In an even more preferred embodiment, F is selected from the group consisting of Texas Red, Tetramethyl rhodamine, MR121.*("*New fluorescent dyes in the red region for biodiagnostics" M. Sauer et al 1995 J. Fluoresc. Vol. 5, pp 247-261*,* or BODIPY-FL 4,4-difluoro-5,7-dimethyl-4-bora-3a,4a- diaza-*s*-indacene-3-propionic acid). In a most preferred embodiment, F is MR121.

A labeled growth hormone secretagogue hereinbefore described can be used for identifying a compound that can bind to a growth hormone secretagogue receptor. Said labeled growth hormone secretagogue can also be used to identify a cellular receptor as a growth hormone secretagogue receptor.

The present invention also provides a process of synthesizing a labeled growth hormone secretagogue which comprises the steps of a) coupling a Cys to the C-terminal amino acid; of R1 and b) reacting the thiol-containing peptide to a fluorescent dye. Preferably, said fluorescent dye is selected from the group consisting of Texas Red, , Tetramethyl rhodamine, MR121.*("*New fluorescent dyes in the red region for biodiagnostics" M. Sauer et al 1995 J. Fluoresc. Vol. 5, pp 247-261*,* or BODIPY-FL (4,4-difluoro-5,7-dimethyl-4-bora-3a,4a-diaza-*s*-indacene-3-propionic acid).

Muccioli et al. (J. Endocrin. Invest. 2001, 24, p.RC7-RC9) discloses a radiolabeled ¹²⁵I-Tyr4-ghrelin. The statement on p. RC8, right column, that the synthetic GHS binding sites are far greater than that of ghrelin receptors indicates that this radiolabeled ghrelin peptide has a high non-specific binding activity.

WO01/92292 dislcoses unlabeled ghrelin analogues. In the binding assay disclosed on p.17, a ³⁵S-labeled growth hormone secretagogue receptor-binding small molecule (not a peptide) is used.

WO01/38355 discloses a motilin-related peptide with a sequence corresponding to ghrelin. A fluorescently labeled peptide without a C-terminal Cys residue is disclosed. Said peptide was only used in in vivo experiments. There is no indication for a use of a fluorescently labeled ghrelin peptide in screenings, including high throughput screening.

The present invention also pertains to an in vitro method for identifying a compound that can bind to a growth hormone secretagogue receptor comprising contacting said compound with a host expressing a growth hormone secretagogue receptor in the presence of the labeled growth hormone secretagogue hereinbefore described, or with a membrane preparation from such a host, and monitoring whether the compound influences the binding of the labeled growth hormone secretagogue hereinbefore described to the growth hormone secretagogue receptor by measuring the fluorescence of the label of the bound labeled growth hormone secretagogue hereinbefore described.

The host, which is not from human embryonic cells, may be a tissue sample, primary cells or cultured cells which either naturally expresses a growth hormone secretagogue receptor, or which are either transiently or stably transfected with a growth hormone secretagogue receptor. Methods of transfecting cells are well known in the art (Sambrook et al., Molecular Cloning: A Laboratory Manual (1989), Cold Spring Harbor Laboratory Press, New York, USA).

Preferably, said method is a high throughput screening method. The method hereinbefore described is sensitive with regard to the pH of the assay buffer used. A pH deviation of 0.2 results in a 50 % loss of binding. Therefore, in a preferred embodiment, the assay buffer used in said method has a pH of 7.2. The peptides of this invention tend to absorb to surfaces. Thus, in a preferred embodiment of this invention, the plastic ware either comprises a nonbinding surface or is blocked with a casein solution. The term "plastic ware" as used herein refers to plates used for the assay, as well as any type of tube used to prepare solutions comprising the peptides of this invention. Preferably, said casein solution comprises 1 % casein. More preferably, blocking is performed over night. In a most preferred embodiment, the blocked plates are washed with buffer before use.

The present invention also provides a method of identifying a cellular receptor as a growth hormone secretagogue receptor comprising contacting a host suspected to express a growth hormone secretagogue receptor with the labeled growth hormone secretagogue hereinbefore described and determining whether binding has occurred.

Furthermore, the present invention pertains to a method for identifying the activity of a compound as a growth hormone secretagogue comprising contacting the compound suspected of having activity as a growth hormone secretagogue with a host expressing a growth hormone secretagogue receptor in the presence of the labeled growth hormone secretagogue hereinbefore described and monitoring whether the compound suspected of having activity as a growth hormone secretagogue influences the binding of the labeled growth hormone secretagogue hereinbefore described to the growth hormone secretagogue receptor.

### Figures:

Fig. 1: Set-up of HTS assay plate

Fig. 2: Competition curves from Zeiss-System, HTS conditions. A) Competition of ghrelin (1-19) MR121 with H6935; b) Competition of ghrelin (1-19) MR121 with hexarelin.

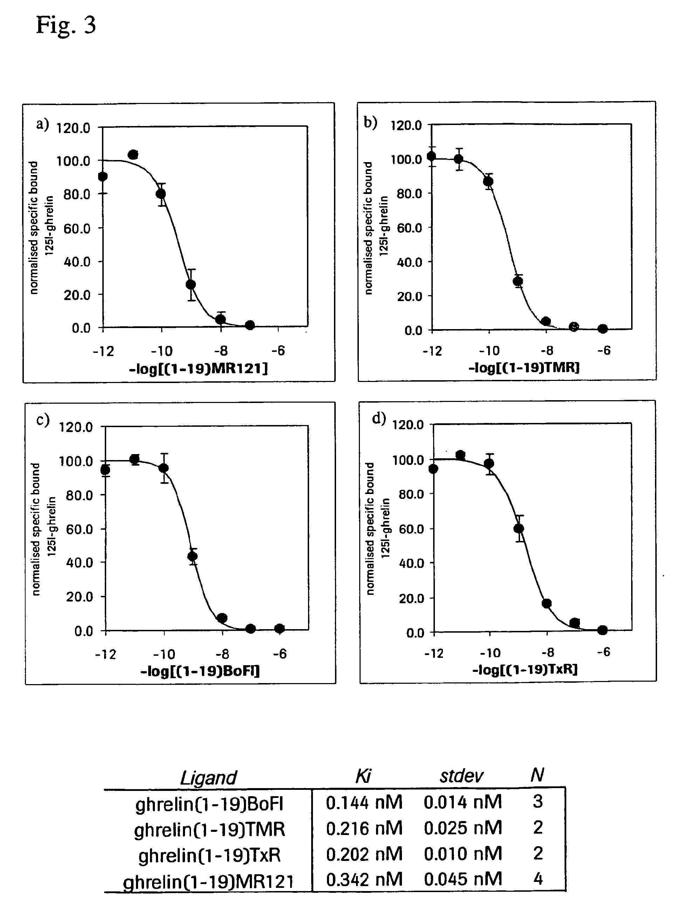

Fig. 3: Affinity of fluorescent analogs, determined in 125I-ghrelin competition assays. A) ghrelin (1-19) MR121; b) ghrelin (1-19) TMR; c) ghrelin (1-19) BoFl (BIODIPY-FL); d) ghrelin (1-19) Texas Red.

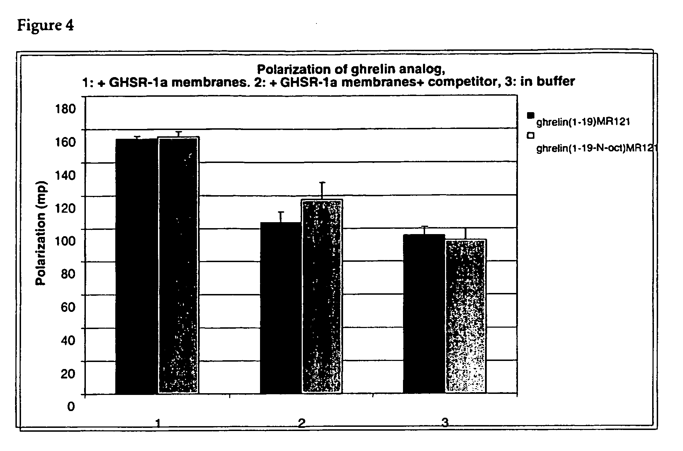

Fig 4: Polarization of fluorescent analogs, determined according to protocol in example 2.2.1 and 2.2.2.

Red (Darker, left hand bar): ghrelin(1-19)MR121, Blue (Lighter, right hand bar): ghrelin(1-19-N-oct)MR121.

1) Fluorescence polarization of Total bound fluorescent ligands (in presence of GHSR-1a membranes); 2) Fluorescence polarization of Free ligand (in presence of GHSR-1a membranes and excess of competitor); 3) Fluorescence polarization of fluorescent ligands in buffer.

### Examples:

### Example 1: Synthesis of the Labeled Peptides

### Example 1.1: Synthesis of ¹Gly-Ser-Ser(octanoyl)-Phe-⁵Leu-Ser-Pro-Glu-His-¹⁰Gln-Arg-Val-Gln-Gln-¹⁵Arg-Lys-Glu-Ser-Cys-NH₂

Continuous-flow solid-phase synthesis was performed on a Pioneer^{™} Peptide Synthesis System, starting fromTenta Gel S RAM resin (0.25 mmol/g (Rapp Polymere GmbH, Tübingen, Germany) according to the method described in "Fmoc Solid Phase Peptide Synthesis: A practical approach" (Oxford University Press 2000) pp 41-74, Eds. W.C. Chan and P.D. White. The base-labile Fmoc group was used for α-amino protection. Side chains were protected with the following protection groups: -Asn(Trt), Glu(OtBu), Ser(tBu), His(Trt), Gln(Trt), His(Trt), Arg(Pbf), Lys(Boc), Cys(Trt). Fmoc-amino acids (4 equiv.) were activated with an equivalent amount of O-(1,2-dihydro-2-oxopyrid-1-yl)-N,N,N',N'-tetramethyluronium tetrafluoroborate (TPTU) and N,N-diisopropylethylamine (Hünig's base). Fmoc deprotection was achieved with 20% piperidine in DMF. The automated synthesis was interrupted after residue ⁴Phe was incorporated in the target sequence. Peptide synthesis was continued semi-manually using a Peptide Synthesizer SP650 (Labortec AG). Side chain unprotected Fmoc-³Ser-OH (0.65 g, 2mmol), TPTU (0.59 g, 2 mmol), Hünig's base (1.03 ml) were added to the peptide resin and coupling was continued for 1 hour in DMF solvent (ninhydrin negative). Octanoylation of the side chain hydroxyl was achieved using caprylic acid (2.0 ml, 12 mmol), N,N'-Diisopropylcarbodiimide, (1.9 ml, 12 mmol) N,N'-dimethylaminopyridine (18 mg, 0.15 mmol) in N-methylpyrrolidine solvent. After 4 hours the reaction mixture was filtered off and synthesis was continued using the standard peptide synthesis protocol (above). ¹Gly- Ser(tBu)-Ser(octanoyl)-Phe-⁵Leu-Ser(tBu)-Pro-Glu(OtBu)- His(Trt)-¹⁰Gln(Trt)-Arg(Pbf)-Val-Gln(Trt)-Gln(Trt)-¹⁵Arg(Pbf)-Lys(Boc)-Glu(OtBu)-Ser(tBu)-Cys(Trt)-NH₂Tenta Gel S-resin (2.0 g) was treated with a mixture (100 ml) of 95% TFA, 2.5% H₂O, 2.5% EDT, 2.5% triisopropylsilane for 5 hours. The reaction mixture was concentrated and poured into diethyl ether and the precipitate was collected by filtration and lyophilized from water. The crude peptide (0.80 g) was purified by preparative RP-HPLC. There was obtained ¹Gly-Ser-Ser(octanoyl)-Phe-⁵Leu-Ser-Pro-Glu-His-¹⁰Gln-Arg-Val-Gln-Gln-¹⁵Arg-Lys-Glu-Ser-Cys-NH₂ (0.18 g, Ion-spray MS analysis (M+2H)²⁺/2 = 1165.4, (M+3H)³⁺/3 = 777.2).

### Example 1.1b: Synthesis of ¹Gly-Ser-Dapa(N-octanoyl)-Phe-⁵Leu-Ser-Pro-Glu-His-¹⁰Gln-Arg-Val-Gln-Gln-¹⁵Arg-Lys-Glu-Ser-Cys-NH₂

### (S)-2-(9H-Fluoren-9-ylmethoxycarbonylamino)-3-octanoylamino-propionic acid; Fmoc-L-Dapa(N-octanoyl)-OH

To a pre-activated mixture containing caprylic acid (1.54 ml, 10 mmol), TPTU (2.8 g, 9.5 mmol) and Hunig's base (3.4 ml, 20 mmol)) in DMF (20 ml) was added a solution of Fmoc-L-Dapa-OH Neosysytem FA04002 8 (3.3 g, 10 mmol) in DMF (10 ml). The reaction mixture was stirred for 1h, concentrated under reduced pressure dissolved in ethyl acetate and washed with 5%/10% KHSO₄/K₂SO₄, brine, dried over Na₂SO₄, filtered and concentrated. Crystallizaton from ethyl acetate/ hexane: 3.7 g, 82% ; MS = 451.4 (MH)⁻.

The peptide synthesis incorporating Fmoc-L-Dapa(N-octanoyl)-OH was performed on a Pioneer^{™} Peptide Synthesis System as described above starting with Tentagel S-NH2 resin (0.55 mmol), yielding purified peptide ¹Gly-Ser-Dapa(N-octanoyl)-Phe-⁵Leu-Ser-Pro-Glu-His-¹⁰Gln-Arg-Val-Gln-Gln-¹⁵Arg-Lys-Glu-Ser-Cys-NH₂ :135 mg; Ion-spray MS: (M+2H)²⁺/2 = 1164.8, (M+3H)³⁺/3 = 776.8).

### Conjugation of peptides to Fluorophores

### Example 1.2: ¹Gly-Ser-Ser(octanoyl)-Phe-⁵Leu-Ser-Pro-Glu-His-¹⁰Gln-Arg-Val-Gln-Gln-¹⁵Arg-Lys-Glu-Ser-Cys(TxR)-NH₂ ;

The thiol-containing peptide above (1.3 mg) was dissolved in 9:1 DMSO: 50 mM phosphatebuffer pH 6 to a final concentration of 2.5 mM. 1.2 equivalent of TxR (Texas Red)-Maleimide (30 mM freshly prepared solution in DMSO) was added and the mixture left to react at room temperature for 10 minutes. The reaction mixture was directly purified by RP-HPLC: 0.17 mg. MS analysis: calculated monoisotopic mass: C136H198N36O39S3= 3055.38; found monoisotopic mass: 3055.35

### Example 1.3: ¹Gly-Ser-Ser(octanoyl)-Phe-⁵Leu-Ser-Pro-Glu-His-¹⁰Gln-Arg-Val-Gln-Gln-¹⁵Arg-Lys-Glu-Ser-Cys(TMR)-NH₂;

The TMR (Tetramethylrhodamine) derivatized peptide was prepared analogously to example 1.2: From1.7 mg starting peptide material was isolated 0.44 mg labeled peptide. MS analysis: calculated monoisotopic mass: C127H185N35O36S = 2808.34; found monoisotopic mass: 2808.40

### Example 1.4: ¹Gly-Ser-Ser(octanoyl)-Phe-⁵Leu-Ser-Pro-Glu-His-¹⁰Gln-Arg-Val-Gln-Gln-¹⁵Arg-Lys-Glu-Ser-Cys(MR121)-NH₂;

The MR121* derivatized peptide was prepared analogously to example 1.2: From 1.5 mg initial peptide material was isolated 0.37 mg labeled peptide. MS analysis: calculated monoisotopic mass: C129H196N37O35S = 2855.44; found monoisotopic mass: 2855.38

### Example 1.5: ¹Gly-Ser-Ser(octanoyl)-Phe-⁵Leu-Ser-Pro-Glu-His-¹⁰Gln-Arg-Val-Gln-Gln-¹⁵Arg-Lys-Glu-Ser-Cys(BODIPY-FL)-NH₂;

The BODIPY-FL (4,4-difluoro-5,7-dimethyl-4-bora-3a,4a- diaza-*s*-indacene-3-propionic acid) derivatized peptide was prepared analogously to example 1.2: From 0.7 mg starting material was isolated 0.20 mg labeled peptide. MS analysis calculated monoisotopic mass: C119H183BF2N36O34S = 2742.4; found monoisotopic mass: 2742.4.

### Example 1.6: ¹Gly-Ser-Dapa(N-octanoyl)-Phe-⁵Leu-Ser-Pro-Glu-His-¹⁰Gln-Arg-Val-Gln-Gln-¹⁵Arg-Lys-Glu-Ser-Cys(MR121)-NH₂;

The MR121* derivatized peptide was prepared analogously to example 1.2: From 0.7 mg initial peptide material was isolated 0.17 mg labeled peptide. MS analysis: calculated monoisotopic mass: C129H197N36O35S = 2854.46; found monoisotopic mass: 2854.49.

* MR-121 is an oxazine fluorescent dye. [see lit.: "New fluorescent dyes in the red region for biodiagnostics" M. Sauer et al 1995 J. Fluoresc. Vol. 5, pp 247-261]

### Example 2: Binding assay

### Example 2.1: Membrane preparation

Human Embryonic Kidney HEK 293 (EBNA) cells were grown in suspension and transfected according to the method previously described (Schlaeger and Christensen, Cytotechnology, 30, 71-83, 1999). The cells were centrifugated for 10 min at 500 rpm, washed once with PBS-0.7 mM EDTA/(4°C) and resuspended in PBS-EDTA-PI (with Protease inhibitor cocktail), at 2 ml/g of cells. Cells were broken with Ultra Turax level green 3 x 15" with 30" breaks on ice. To remove debris the suspension was centrifugated in a Sorvall SS34 rotor for 20min at 2'000 rpm. The supernatant was collected and centrifugated for 40 min at 20'000 rpm. The pellet was resuspended in PBS-EDTA. Receptor density was verified with saturation binding assay using T- MK 0677 to be 4.9 pmol/mg protein.

### Example 2.2: FP-assay

### Example 2.2.1 Assay development

GHSR-la-containing cell-membranes was diluted in FP-buffer: 25 mM Hepes, 5 mM MgCl₂, 1 mM CaCl₂, 4% PEG, 0.1% BSA (fraction V) to a to a final volume of 0.5-1 ml, passed through a 0.4 mm syringe and sonicated 4 times 20 pulses while kept on ice. 10 nM solution of MR121-labeled ghrelin (tracer) and 20 x concentrated solutions of competitor in FP-buffer were prepared. To determine polarization of receptor bound tracer, three samples of 180 ul were prepared: Total bound: Membranes + tracer, Free ligand: membranes + tracer + competitor, Fluorescence background: membranes + buffer. 3 x 50 µl of each sample was transferred to assay-plates right after mixing.

### Example 2.2.2 HTS protocol for FP-ghrelin Competition Binding Assay

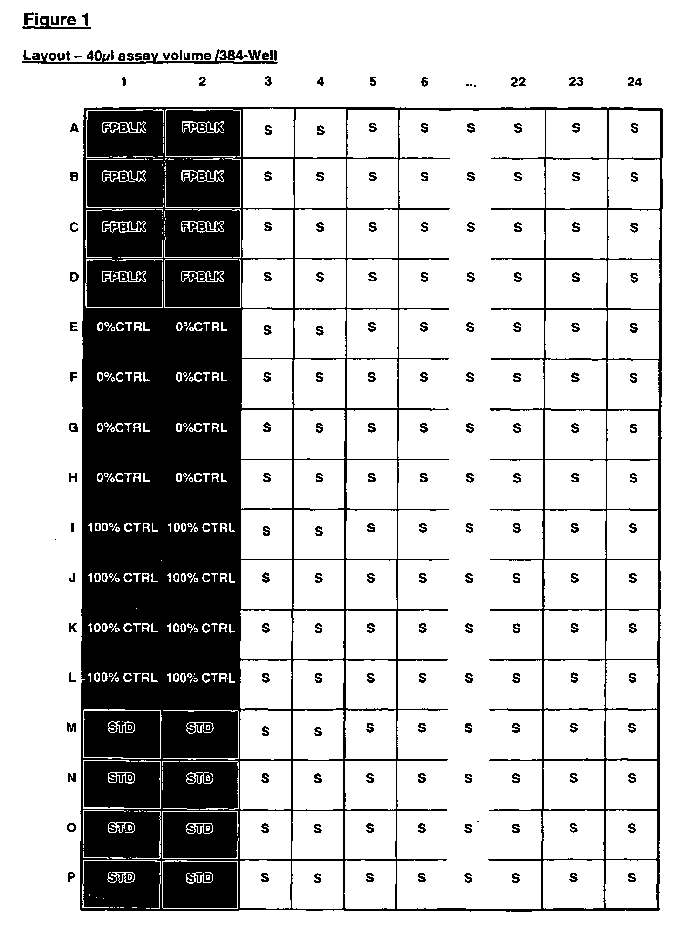

Figure 1. shows a possible layout of a 384 well plate for High Throughput Screening. For one round of screening, 119 Sample Plates and 1 DMSO Plate (membranes p20F1+p22F1) were used. The following is a representative non-limiting example of a HTS protocol with MR121 as fluorochrome. For the assay, Costar 384 well UV plates with non-binding surface were used. The following assay buffer was used: 25mM Hepes pH 7.2, 5mM MgCl₂, 1mM CaCl₂, 4% polyethylene glycol, and 0.1% BSA (Fract. V) was added fresh every week. Receptors were provided as follows: Membranes were isolated as described in example 2.1. Final assay concentration was 1.4 nM of GHSR-1a as determined by 125 I-ghrelin saturation binding. Typical values for the membrane stock are: Bmax = 6 fmol/µg; Protein Concentration= 50µg/µl.

To avoid sedimentation membranes need to be pushed through a needle (3x / 0.4mm) and sonicated "Branson sonifier 250" set to intensity level 3-4, 4x20 pulses separated by 30 sec pause. Membranes were kept on ice during sonication. Membranes were diluted to an endconcentration of 1.4 nM of receptor.

The tracer ghrelin(1-19)[K19MR121] was diluted in buffer from 1µM DMSO stock. Final assay concentration was 0.5nM. Since the peptide tends to adsorb to surfaces, the plastic ware used for the diluted peptide solution either comprised a nonbinding surface or was blocked overnight with a 1% casein solution, and then washed with buffer before use.

The following steps were used for high throughput screening:
1) 30µl of 1.33x membrane solution were added to all wells of the assay plate.
2) 4.4µl of buffer were transferred to "FPBLK" /"100% control" wells and 4.4µl of reference compound solution were transferred to "STD" and "0% control" wells from a reservoir plate to the assay plate
3) 10µl of water with 0% DMSO were added to columns 3 to 24 of the compound storage plate containing 1µl of 2 mM compounds (Endconc. 20µM).
4) The contents of the storage plate were mixed.
5) 4.4µl of diluted compounds were transferred from the storage plate to an assay plate.
6) The contents of the assay plate were mixed five times and then incubated for 30 min at 24°C.
7) 5.6µl of 7.143x tracer solution were added to the assay plate except to wells A1 to D2 (wells labeled "FPBLK" in Fig. 1) to which 5.6µl of buffer were added.
8) The content of the assay plate was mixed five times.
9) 30µl of the solution in each well were transferred from the assay plate to the read-out plate (Corning UV non-binding surface).
10)The readout plate was incubated for 10 min. at RT.
11)MR121 fluorescence was read from the read-out plate at 650-695 nm (5 s, Focus To Bottom 1 mm, xy scan 0.5 mm), with settings at parallel (∥) and crossed (⊥) polarization, using a Zeiss plate::vision microtiter plate reader.

## Claims

1. A labeled growth hormone secretagogue of the formula
R1-Cys-F
wherein R1 is a peptide sequence derived from the ghrelin polypeptide sequence of Seq ID No. 1, Cys is attached to the C-terminal amino acid of R1 and F is a fluorochrome.

2. The labeled growth hormone secretagogue of claim 1, wherein R1 is Seq ID No. 2.

3. The labeled growth hormone secretagogue of claim 1, wherein R1 in octanoylated.

4. The labeled growth hormone secretagogue of claim 1, wherein R1 is N-octanoylated.

5. The labeled growth hormone secretagogue of claim 1, wherein R1 is Gly-Ser-Ser(octanoyl)-Phe-⁵Leu-Ser-Pro-Glu-His-¹⁰Gln-Arg-Val-Gln-Gln-¹⁵Arg-Lys-Glu-Ser.

6. The labeled growth hormone secretagogue of claim 1, wherein R1 is ¹Gly-Ser-Dapa(N-octanoyl)-Phe-⁵Leu-Ser-Pro-Glu-His-¹⁰Gln-Arg-Val-Gln-Gln-¹⁵Arg-Lys-Glu-Ser.

7. The labeled growth hormone secretagogue of claims 1 to 6, wherein F is selected from the group consisting of TxR, TMR, MR121 or BODIPY-FL.

8. In vitro use of a labeled growth hormone secretagogue of any one of claims 1 to 7 for identifying a compound that can bind to a growth hormone secretagogue receptor.

9. The use of claim 8 being a method comprising the steps of:
a) contacting said compound with a membrane isolated from a cell expressing a growth hormone secretagogue receptor in the presence of the labeled growth hormone secretagogue of any one of claims 1 to 7 in an assay buffer;
b) washing said membrane to remove unbound labeled growth hormone secretagogue; and
c) monitoring whether the compound influences the binding of the labeled growth hormone secretagogue of any one of claims 1 to 7 to the growth hormone secretagogue receptor by measuring the fluorescence of said membranes.

10. The use of claim 9, wherein said method is a high throughput screening method.

11. The use of claim 9 or 10, wherein said assay buffer has a pH of 7.2.

12. A process of synthesizing a labeled growth hormone secretagogue according to claims 1 to 7 which comprises the steps of
a) coupling a Cys to the C-terminal amino acid; and
b) reacting the thiol-containing peptide to a fluorescent dye.

13. An in-vitro method of identifying a cellular receptor as a growth hormone secretagogue receptor comprising contacting a host suspected to express a growth hormone secretagogue receptor with the labeled growth hormone secretagogue of any one of claims 1 to 7 and determining whether binding has occurred.

## Patentansprüche

1. Markiertes Wachstumshormon-Sekretagogum der Formel
R1-Cys-F,
worin R1 eine Peptidsequenz, abgeleitet von der Ghrelinpolypeptidsequenz mit Seq ID Nr. 1, ist, Cys an die C-terminale Aminosäure von R1 gebunden ist und F ein Fluorochrom ist.

2. Markiertes Wachstumshormön-Sekretagogum nach Anspruch 1, wobei R1 Seq ID Nr. 2 ist.

3. Markiertes Wachstumshormon-Sekretagogum nach Anspruch 1, wobei R1 octanoyliert ist.

4. Markiertes Wachstumshormon-Sekretagogüm nach Anspruch 1, wobei R1 N-octanoyliert ist.

5. Markiertes Wachstumshormön-Sekretagogum nach Anspruch 1, wobei R1 ¹Gly-Ser-Ser(octanoyl)-Phe-⁵Leu-Ser-Pro-Glu-His-¹⁰Gln-Arg-Val-Gln-Gln-¹⁵Arg-Lys-Glu-Ser ist.

6. Markiertes Wachstumshormon-Sekretagögum nach Anspruch 1, wobei R1 ¹Gly-Ser-Dapa(N-octanoyl)-Phe-⁵Leu-Ser-Pro-Glu-His-¹⁰Gln-Arg-Val-Gln-Gln-¹⁵Arg-Lys-Glu-Ser ist.

7. Markiertes Wachstumshormon-Sekretagogum nach-den Ansprüchen 1 bis 6, wobei F aus der Gruppe ausgewählt ist, bestehend aus TxR, TMR, MR121 oder BODIPY-FL.

8. In-Vitro-Verwendung eines markierten Wachstumshormon-Sekretagogums nach einem der Ansprüche 1 bis 7 zum Identifizieren einer Verbindung, die an einen Wachstumshormon-Sekreteagogum-Rezeptor binden kann.

9. Verwendung nach Anspruch 8, die ein Verfahren ist, umfassend die Schritte:
a) Kontaktieren der Verbindung mit einer Membran, die aus einer Zelle, welche einen Wachstumshormon-Sekretagogum-Rezeptor exprimiert, isoliert wurde, in Gegenwart des markierten Wachstumshormon-Sekretagogums nach einem der Ansprüche 1 bis 7 in einem Assay-puffer;
b) Waschen der Membran zur Entfernung des ungebundenen markierten Wachstumshormon-Sekretagogums und
c) Überwachen, ob die Verbindung die Bindung des markierten Wachstumshormon-Sekretagogums nach einem der Ansprüche 1 bis 7 an den Wachstumshormon-Sekretagogum-Rezeptor beeinflußt, durch Messen der Fluoreszenz der Membranen.

10. Verwendung nach Anspruch 9, wobei das Verfahren ein Hochdurchsatz-Screening-Verfahren ist.

11. Verwendung nach Anspruch 9 oder 10, wobei der Assaypuffer einen pH von 7,2 aufweist.

12. Verfahren zum Synthetisieren eines markierten Wachstumshormon-Sekretagogums nach den Ansprüchen 1 bis 7, umfassend die Schritte:
a) Verknüpfen eines Cys mit der C-terminalen Aminosäure und
b) Umsetzen des Thiol-enthaltenden Peptids mit einem Fluoreszenzfarbstoff.

13. In-Vitro-Verfahren zum Identifizieren eines zellulären Rezeptors als ein Wachstumshormon-Sekretagogum-Rezeptor, umfassend das Kontaktieren eines Wirts, von dem angenommen wird, daß er einen Wachstumshormon-Sekretagogum-Rezeptor exprimiert, mit dem markierten Wachstumshormon-Sekretagogiun nach einem der Ansprüche 1 bis 7 und Bestimmen, ob eine Bindung auftrat.

## Revendications

1. Sécrétagogue d'hormone de croissance marqué de formule
R1-Cys-F
dans laquelle R1 est une séquence peptidique dérivée de la séquence polypeptidique de la ghréline de SEQ ID NO: 1, Cys est lié à l'aminoacide C-terminal de R1 et F est un fluorochrome.

2. Sécrétagogue d'hormone de croissance marqué selon la revendication 1, dans lequel R1 est SEQ ID NO: 2.

3. Sécrétagogue d'hormone de croissance marqué selon la revendication 1, dans lequel R1 est octanoylé.

4. Sécrétagogue d'hormone de croissance marqué selon la revendication 1, dans lequel R1 est N-octanoylé.

5. Sécrétagogue d'hormone de croissance marqué selon la revendication 1, dans lequel R1 est ¹Gly-Ser-Ser(octanoyl)-Phe-⁵ Leu-Ser-Pro-Glu-His-¹⁰Gln-Arg-Val-Gln-Gln-¹⁵Arg-Lys-Glu-Ser.

6. Sécrétagogue d'hormone de croissance marqué selon la revendication 1, dans lequel R¹ est ¹Gly-Ser-Dapa(N-octanoyl)-Phe-⁵Leu-Ser-Pro-Glu-His-¹⁰Gln-Arg-Val-Gln-Gln-¹⁵Arg-Lys-Glu-Ser.

7. Sécrétagogue d'hormone de croissance marqué selon les revendications 1 à 6, dans lequel F est choisi dans le groupe constitué par TxR, TMR, MR121 ou BODIPY-FL.

8. Utilisation *in vitro* d'un sécrétagogue d'hormone de croissance marqué selon l'une quelconque des revendications 1 à 7 pour l'identification d'un composé capable de se lier à un récepteur de sécrétagogue d'hormone de croissance.

9. Utilisation selon la revendication 8, qui est un procédé comprenant les étapes selon lesquelles:
a) on met ledit composé en contact avec une membrane isolée à partir d'une cellule exprimant un récepteur de sécrétagogue d'hormone de croissance en présence du sécrétagogue d'hormone de croissance marqué selon l'une quelconque des revendications 1 à 7 dans un tampon d'analyse;
b) on lave ladite membrane pour séparer le sécrétagogue d'hormone de croissance marqué non lié; et
c) on contrôle si le composé influence la liaison du sécrétagogue d'hormone de croissance marqué selon l'une quelconque des revendications 1 à 7 au récepteur de sécrétagogue d'hormone de croissance en mesurant la fluorescence de ladite membrane.

10. Utilisation selon la revendication 9, dans laquelle ledit procédé est un procédé de criblage à haut débit.

11. Utilisation selon la revendication 9 ou 10, dans laquelle ledit tampon d'analyse a un pH de 7,2.

12. Procédé de synthèse d'un sécrétagogue d'hormone de croissance marqué selon les revendications 1 à 7, qui comprend les étapes de:
a) couplage d'une Cys à l'aminoacide C-terminal; et
b) réaction du peptide contenant un thiol avec un colorant fluorescent.

13. Procédé d'identification *in vitro* d'un récepteur cellulaire en tant que récepteur de sécrétagogue d'hormone de croissance, dans lequel on met un hôte suspecté d'exprimer un récepteur de sécrétagogue d'hormone de croissance en contact avec le sécrétagogue d'hormone de croissance marqué selon l'une quelconque des revendications 1 à 7 et on détermine si une liaison s'est produite.


### Sequence listing

<110> F. Hoffmann-La Roche AG
<120> Fluorescent labeled ghrelin
<130> 22229
<160> 2
<170> PatentIn version 3.2
<210> 1
   <211> 117
   <212> PRT
   <213> Homo sapiens
<220>
   <221> ghrelin precursor
   <222> (1)..(117)
<220>
   <221> ghrelin
   <222> (24)..(117)
   <223> mature protein
<220>
   <221> ghrelin
   <222> (26)..(26)
   <223> octanoyl derivative residue
<400> 1
   Met Pro Ser Pro Gly Thr Val Cys Ser Leu Leu Leu Leu Gly Met Leu
   1               5                   10                  15
   Trp Leu Asp Leu Ala Met Ala Gly Ser Ser Phe Leu Ser Pro Glu His
               20                  25                  30
   Gln Arg Val Gln Gln Arg Lys Glu Ser Lys Lys Pro Pro Ala Lys Leu
           35                  40                  45
   Gln Pro Arg Ala Leu Ala Gly Trp Leu Arg Pro Glu Asp Gly Gly Gln
       50                  55                  60
   Ala Glu Gly Ala Glu Asp Glu Leu Glu Val Arg Phe Asn Ala Pro Phe
   65                  70                  75                  80
   Asp Val Gly Ile Lys Leu Ser Gly Val Gln Tyr Gln Gln His Ser Gln
                   85                  90                  95
   Ala Leu Gly Lys Phe Leu Gln Asp Ile Leu Trp Glu Glu Ala Lys Glu
               100                 105                 110
   Ala Pro Ala Asp Lys
           115
<210> 2
   <211> 18
   <212> PRT
   <213> Homo sapiens
<220>
   <221> ghrelin(1-19)
   <222> (1)..(18)
<220>
   <221> ghrelin(1-19)
   <222> (3)..(3)
   <223> octanoyl derivative
<400> 2
   Gly Ser Ser Phe Leu Ser Pro Glu His Gln Arg Val Gln Gln Arg Lys
   1               5                   10                  15
   Glu Ser